(19) Europäisches Patentamt
European Patent Office
Office européen des brevets

(11) Publication number: **0 187 680 B1**

(12) **EUROPEAN PATENT SPECIFICATION**

(45) Date of publication of patent specification: **28.04.93**

(51) Int. Cl.5: **C12P 7/40**, C12P 13/04, C12P 17/12, C12N 13/00

(21) Application number: **86100305.1**

(22) Date of filing: **11.01.86**

(54) **Process for producing organic acids by use of microorganisms.**

(30) Priority: **11.01.85 JP 2766/85**

(43) Date of publication of application:
**16.07.86 Bulletin 86/29**

(45) Publication of the grant of the patent:
**28.04.93 Bulletin 93/17**

(84) Designated Contracting States:
**DE FR GB**

(56) References cited:
**US-A- 3 880 717**
**US-A- 3 940 316**

**CHEMICAL ABSTRACTS, vol. 103, no. 1, July 1985, Columbus, OH (US); no. 5046z***

(73) Proprietor: **NITTO KAGAKU KOGYO KABUSHIKI KAISHA**
**5-1, Marunouchi 1-chome**
**Chiyoda-Ku Tokyo-To(JP)**

Proprietor: **MITSUBISHI RAYON KABUSHIKI KAISHA**
**3-19, Kyobashi 2-Chome Chuo-ku**
**Tokyo-To(JP)**

(72) Inventor: **Enomoto, Kanehiko**
**36-5-106, Kyodo 5-Chome Setagaya-Ku**
**Tokyo-To(JP)**
Inventor: **Sato, Yoshiaki**
**138-21, Setogaya-Cho Hodogaya-Ku**
**Yokohama-Shi Kanagawa-Ken(JP)**
Inventor: **Nakashima, Yasutaka**
**2-6-206, Kurokawa 3-Chome**
**Otake-Shi Hiroshima-Ken(JP)**
Inventor: **Fujiwara, Atsushi**
**245-1, Kamihoshikawa Hodogaya-Ku**
**Yokohama-Shi Kanagawa-Ken(JP)**
Inventor: **Doi, Toshiaki**
**298-8, Setogaya-Cho Hodogaya-Ku**
**Yokohama-Shi Kanagawa-Ken(JP)**

(74) Representative: **Reichel, Wolfgang, Dipl.-Ing. et al**
**Reichel und Reichel Parkstrasse 13**
**W-6000 Frankfurt am Main 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.5x/3.0.1)

## Description

### BACKGROUND OF THE INVENTION

### Field of the Art

This invention relates to a process for hydrating and hydrolyzing a nitrile compound by the action of microorganisms (hereinafter including crushed cells thereof, liquid extracts and crude enzyme solution therefrom, etc) having nitrilase activity to convert the nitrile compound into the corresponding organic acid. More particularly, this invention relates to the process for producing a carboxylic acid by irradiation with light of the microorganisms with good yield, space time yield and productivity per bacterial cells (i.e. the amount of organic acids to be produced per unit amount of bacterial cells used).

Recently there have been developed processes for carrying out chemical reactions by the use of microorganisms or enzymes obtained therefrom. In general, the reactions by means of microorganisms or enzymes are advantageous in that consumption of energy for the reactions is small because the reaction can be carried out at room temperature and atmospheric pressure and that the desired products of high purity are easily obtained because the selectivity of the reaction to yield the desired products is very high. On the other hand, there is room for improvement with respect to the reaction activity, the life time of microorganisms or enzymes used as catalysts. Especially, there are problems when the velocity of the desired reaction (i.e. reaction activity) is low under its optimum conditions and especially at its optimum temperature and/or pH. In such a case, the productivity per bacterial cells used is reduced because large capacity reactors are required owing to low space yield, reaction takes a longer time owing to a slow reaction velocity, and also the reaction activity is lowered.

Thus, it is fundamentally important to realize a high reaction activity of the microorganisms or enzymes to be used. Such reaction activity is a main economical factor of industrial production.

### Prior Art

Processes for hydrating and hydrolyzing nitrile compounds to produce the corresponding organic acids by the action of microorganisms having enzymatic activity which hydrates and hydrolyzes a nitrile compound into the corresponding organic acid (i.e. nitrilase activity and amidase activity) are described in Japanese Patent Publication No. 15120/83 Specification. Microorganisms play an important role in this reaction, and several microorganisms are disclosed in the specification referred to above.

### Problems

It has been found by the present inventors that the nitrilase activity cannot be exhibited sufficiently when a large metal reaction apparatus is used in order to carry out hydration and hydrolysis reactions of nitrile compounds in an industrial scale by utilizing the nitrilase activity of these microorganisms. It has been uncertain whether these phenomena are caused by the microorganisms themselves or the material and structure of the reaction apparatus or other reasons. Thus it was necessary to solve these problems for industrial operation of a microbiological process for producing organic acids.

### SUMMARY OF THE INVENTION

It is an object of the present invention to solve the above mentioned problems. This object is achieved by irradiation with light of microorganisms to be used.

According to the present invention, there is presented an improvement in a process for hydrating and hydrolyzing a nitrile compound by the action of a microorganism having nitrilase activity to convert the nitrile compound into the corresponding organic acid, and the improvement comprises conducting the reaction under the conditions wherein:

(a) the microorganism having nitrilase activity is positive Gram staining;

(b) the microbial cells are allowed to accept light energy of at least about 2 x $10^{-3}$ $\mu$mol of photons/g microbial cells second before termination of the hydration reaction, and

(c) the hydration reaction is carried out in a vessel composed at least partly of a material which does not permit transmission of light therethrough.

Thus, in the hydration and hydrolysis reactions of a nitrile compound by the action of a microorganism having nitrilase activity according to the present invention, (i) the hydration reaction which does not

substantially proceed in a reaction vessel composed of a non-light-transmitting material where light is substantially absent can proceed by irradiation of the microorganisms with light, and (ii) the hydration reaction which proceeds to some extent in a reaction vessel composed partly of a non-light-transmitting material where some amount of light is present can proceed substantially faster by the irradiation with light.

That the reaction of producing organic acids from the corresponding nitrile compounds is promoted by irradiation with light may be explained as follows.

The production of organic acids according to the present invention is brought about by the following two successive reactions caused by the microorganisms having nitrilase activity.

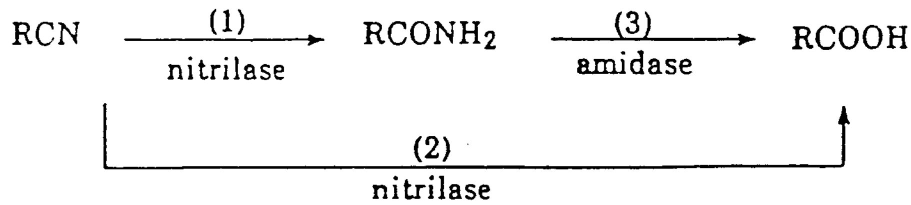


In the above reaction, the effect of promoting the reaction by irradiation with light was observed in reactions (1) and (2), but not at all observed in reaction (3). It has already been found by the present inventors that reaction (1) is promoted by irradiation with light, which is disclosed in Japanese Patent Application No. 125588/83 Specification, and the present invention was made as an improvement thereof. As a result of intensive study based on the idea that the promotion of reactions (1) and (2) by irradiation with light would also be effective in the production of organic acids by biochemical hydrolysis reaction, it has been found by the inventors that the velocity of reaction to produce organic acids from the corresponding nitrile compounds is accelerated and thus the productivity can be enhanced by the irradiation. On the basis of this finding, the present inventors have arrived at this invention. Therefore, as microorganisms to be used in the present method, such microorganisms as have enzyme activity to all of the above reactions (1), (2) and (3) are very useful.

It is well known that useful substances are produced according to microbiological methods including the use of enzymes. However, enhancement of the activity of microorganisms or their enzymes by irradiation with electromagnetic waves such as light is not believed to have been well known. On the contrary, it is generally believed that the irradiation with light is often harmful. Thus, it can be said that the effect of irradiation with light which is exhibited only in the above mentioned case has not been expected in view of the usual results of light on microorganism.

## DETAILED DESCRIPTION OF THE INVENTION

### Microorganisms to be used

As described above, it is known that a nitrile compound is converted to the corresponding organic acid by the action of microorganisms having nitrilase and amidase activity. The present inventors have found that all of the microorganisms having nitrilase activity have also amidase activity and many of them can be used as microorganisms to carry out the present invention. The microorganisms belonging to the genuses described in Japanese Laid-Open Patent Application Nose 53586/75 and 129190/79 Specifications generally have improved activity by irradiation with light and thus are available.

It is the common characteristics of the microorganisms belonging to these genuses that (i) the microorganisms have positive Gram-staining property, (ii) they generally contain phospholipid, (iii) their cell membranes are thick and thus exhibit some resistance to the permeation therethrough with substances, and (iv) crushing of the membranes is not easy in order to take out intra-cellular enzymes.

It is of interest to note that the light irradiation effect according to the present invention owes to apparent increase in nitrilase activity by enhancement of the velocity of substrate (nitrile) and/or product (organic acid) permeating through cell membranes, and to enhancement of nitrilase or enzyme activity itself by the irradiation irrespective of the permeation through cell membranes.

The microorganisms to be used in the present invention are Gram-positive bacteria having nitrilase activity. Specific examples of such microorganisms include the bacteria belonging to the following genuses:

(a) Corynebacterium;

(b) Nocardia;

(c) Bacillus;

(d) Bacteridium;
(e) Micrococcus; and
(f) Brevibacterium.

Specific strains belonging to these genuses include: Corynebacterium --- N-771 strain (FERM P 4445 deposited at a Japanese depository, Fermentation Research Institute, Agency of Industrial Science and Technology, 1,3-Higashi 1-chome, Yatabe-machi, Tsukuba-gun, Ibarakiken, Japan, which depository is herein called FRI, on May 30, 1978) and N-774 strain (FERM P 4446 deposited at FRI on May 30, 1978); Nocardia genus --- N-775 strain (FERM P 4447 deposited at FRI on May 30, 1978), these strains being described in Japanese Patent Publication No. 17918/81 and No. 38118/81 Specifications; and the strains of Bacillus genus and others which are described in Japanese Patent Publication No. 15120/1983 Specification and reported to have been deposited at CBS*) and FRI. The bacteriological properties of these strains are described in these publicly known specifications.

## Nitrile compounds and amide compounds

The nitrile compounds are represented by the general formula $R\text{-}(CN)_n$ and encompass broad ranges of compounds. The compounds include mononitriles when $n=1$ and polynitriles when $n \geqq 2$. The R is a saturated or unsaturated hydrocarbon residue which has a variety of numbers of carbon atom and also has a straight, branched or cyclic chain. The hydrocarbon residue can further contain amino group, hydroxyl group, a halogen, carboxyl group or other substituents which are compatible with the microorganism used.

Examples of preferable nitrile compounds include acetonitrile, propionitrile, n-butyronitrile, isobutyronitrile, n-valeronitrile, acrylonitrile, methacrylonitrile, benzonitrile, cyanopyridine, malononitrile, succinonitrile, fumaronitrile, chloroacetonitrile, $\beta$-hydroxypropionitrile, aminoacetonitrile and $\beta$-aminopropionitrile.

As a result of experiments with many nitrile compounds as shown in the following Examples, it has been found that the nitrilase activity is enhanced without exception by the irradiation with light. Thus, it is said that the present invention can be generally established with respect to nitrile compounds.

The resulting hydration and hydrolysis product is the corresponding acid compound wherein the CN group of the starting nitrile compound is hydrated and hydrolysed into a COOH group.

From the viewpoint of usefulness of the products, important at least at present will be the production of acrylic acid from acrylonitrile, nicotinic acid from cyanopyridine, and amino acids from aminonitriles.

## Hydration reaction and hydrolysis

It is known to carry out the hydration and hydrolysis reactions of a nitrile compound under the action of microorganisms having nitrilase and amidase activities. In the present invention, the hydration can be conducted in any desired embodiment so far as the object of the invention is not impaired. It is to be noted that no alteration in the hydration and hydrolysis reactions themselves has been observed by irradiation with light.

The method of the hydration and hydrolysis reactons generally comprises contacting the starting nitrile compound with the bacterial cells in an aqueous medium for a predetermined period of time.

The bacterial cells can be in the form of a liquid culture; intact bacterial cells, crushed cells, liquid extract and crude enzyme solution separated from the culture medium; or those supported on or immobilized in a carrier. The preferred form of the bacterial cells are intact cells or those immobilized in a polymer gel such as crosslinked polyacrylamide gel or crosslinked polyvinyl alcohol.

The concentrations of the substrate (nitrile) and the bacterial cells in the aqueous medium can be suitably selected, and are generally in a concentration of the substrate ranging from about 0.01 to about 10% by weight and in a concentration of bacterial cells ranging from about 0.01 to about 5% by weight. The reaction temperature, reaction pH and other reaction conditions can be determined, depending on the type of the microorganisms to be used. Normally, the reaction temperature is in the range of about 0 to about 20°C, and the pH is in the vicinity of about 7. A suitable buffer agent can be used in order to maintain the pH at a predetermined level. For the reaction with good efficiency, it is effective to maintain the pH in the range of 8.0 to 8.5 in the early stage of reaction (reaction (1)).

Incidentally, preparation of the bacterial cells to be used in the reaction is readily conducted by culturing the microorganism under suitable conditions. The bacterial cells separated from a culture medium and used for the hydration and hydrolysis reactions are generally under non-proliferating conditions.

*) CBS=Centraal Bureau Voor Schimmelcultures, Baarn, Netherlands

Irradiation with light

(1) Light

The irradiation with light is conducted onto the bacterial cells which are in the stage before termination of the hydration and hydrolysis reactions. The term "before termination of hydration and hydrolysis reactions" means any point of time before termination of the reactions, wherein the termination does not always mean the conversion of 100%. Since the irradiation with light is conducted onto the bacterial cells, the stage before termination of hydration reaction also includes the point of time before contacting the cells with a nitrile compound. Accordingly, the irradiation can be conducted before and/or after contacting bacterial cells with a nitrile compound.

The irradiation can thus be conducted solely before the cells are contacted with a nitrile. When the bacteria cells irradiated with light are contacted with a nitrile compound in the absence of light, however, especially when the intact bacterial cells are used the effect of enhancing the nitrilase activity by the irradiation with light cannot be continued sufficiently, although the effect is revived by irradiation with light. Thus, in a preferred embodiment of the present invention, the bacterial cells are contacted with a nitrile compound and then irradiated with light. Specifically, the irradiation is conducted, for example, before and after contact of the bacterial cells with a nitrile compound.

The "light" to be irradiated according to the present invention can have any wavelength as far as the effect of irradiation is exhibited. However, as readily understood in view of the light being a region of electromagnetic waves, the effect is small when the light is in a longer wavelength region emitted from a lower energy atomic level. On the other hand, when the wavelength is too short, its energy is too large and the bacterial cells or the molecules (arrangement) of enzymes will be destroyed. Thus, the bacterial cells will lose their enzymatic activity and finally result in so-called sterilization.

In accordance with the inventors' research in the broad range of wavelength regions, the light having a long wavelength over 800 nm has no abnormal effect on bacterial cells, but its effect of enhancing the enzymatic activity is not remarkable. When the light has a short wavelength less than 100 nm, the effect of enhancing the activity can be exhibited in a short period of time, but the cells irradiated result in unrecoverable decrease in their activity. Thus, the light preferred in the present invention has a wavelength of 100 to 800 nm, and the light having a wavelength of about 300 to about 450 nm is more preferred. Specifically, such light can be obtained, for example, from natural light such as sunlight and artificial light sources such as a light for sterilization in a wavelength range of 200 to 300 nm, a blacklight lamp in a wavelength range of 300 to 400 nm, and a light for general irradiation in a wavelength range of 400 to 800 nm.

The irradiation with light can be carried out with any strength or amount as far as the effect of enhancing the activity is observed. More specifically, the irradiationn should be conducted so that the bacterial cells receive light energy of at least about $2 \times 10^{-3}$ $\mu$E/g cells•second, preferably at least about $5 \times 10^{-3}$ $\mu$E/g cells•second. Incidentally, the use of immobilized cells sometimes decreases the reaction velocity by from about 1/2 to about 1/3 in comparison with the use of living cells at the same concentration. In this case, the irradiation requires light energy about 2 to 3 times as large as the above mentioned value. As to the light energy, the unit $\mu$mol of photons ("$\mu$E") is the amount of light energy expressed by mol of photons x $10^{-6}$ or E (einstein) x $10^{-6}$ which corresponds to the energy of photons equivalent to the numbers of 1 mole molecules. The unit "g cells" is the weight expressed in gram of dried bacterial cells. The "second" is a period of irradiation expressed in second.

The amount of light energy, namely at least ca. $2 \times 10^{-3}$ $\mu$mol of photons/g cells•second is larger than that of the environmental light present in a reaction vessel or other reaction apparatus of an ordinary industrial scale, i.e., larger than that which is given by room light and/or scattered sunlight into a room. More particularly, according to the inventors' experiment, the luminous intensity at industrial production sites is normally about 100 luxes. At such intensity of light, the amount of light energy received by a reaction vessel as large as about 250 liters of capacity will be short of the above defined $2 \times 10^{-3}$ $\mu$mol of photons/g cells•second, even when the concentration of bacterial cells is as low as 100 ppm and the upper portion of the reaction vessel is fully exposed to light.

Thus, in industrial production sites, it is difficult to satisfy the required amount of light energy even under the conditions accessible to light. When the irradiation with light is conducted according to the present invention, the room light if any given to the bacterial cells is included in the total amount of the light energy of at least about $2 \times 10^{-3}$ $\mu$mol of photons/g cells•second.

In order to obtain the required reaction velocity in the process of the present invention, the irradiation is carried out, for example, for about 1 hour at the lowest light energy of $2 \times 10^{-3}$ $\mu$mol of photons/g

cells•second.

(2) Irradiation

The irradiation can be conducted in any way, as far as bacterial cells before or in contact with a nitrile compound can successively or intermittently receive the required amount of light energy from a light source for irradiation.

The term "a light source for irradiation of bacterial cells" means a light source placed inside or outside of the reaction apparatus for irradiation of the bacterial cells with light, and also includes some light such as scattered sunlight and/or room light which may come into the reaction apparatus surrounding the reaction apparatus. Incidentally, when one makes the room where a reaction apparatus is installed excessively or extraordinarily bright so that significant light energy from the room light can come into the reaction apparatus, such a light source should be understood as a light source for irradiation according to the present invention.

More specifically, the irradiation with light can be conducted by, for example, (a) a light source placed at an upper space over a vessel containing bacterial cells (i.e. space over bacterial cells or over an aqueous suspension thereof), (b) irradiation through a window equipped at a top, side or bottom portion of the reaction vessel with an outside light source, (c) irradiation by means of a lamp placed within a liquor in the reaction vessel so that the vessel can also be used as a photochemical reactor, and (d) other methods. As necessary and if possible, reaction liquor is taken out of a vessel (especially a reaction vessel) containing bacterial cells, and the liquor containing the cells is then irradiated in another vessel by a method as described above. The vessel for irradiation in this case can, for example, comprise one or more transparent glass tubes.

The effect of light irradiation according to the present invention can be markedly exhibited, when a large reaction vessel of industrial scale is used wherein the incident light energy from an ordinary room light is short of the required amount of light energy. Such a large reaction vessel is normally made of a non-light-transmitting material and especially of a metal material in its substantial portions. In such a non-light-transmitting reaction vessel, the light energy obtained from ordinary illumination through a window or a cover of the vessel is very small. Thus, positive irradiation with light is indispensable. Incidentally, the term "reaction vessel" herein means a portion of reaction apparatus wherein at least major hydration and hydrolysis reactions are carried out.

## Experimentation

### Example 1

Washed bacterial cells of N-774 strain (Corynebacterium) were prepared by aerobically culturing the strain in a medium (pH 7.2) comprising glucose 1%, peptone 0.5%, yeast extract 0.3%, malt extract 0.3%, and ferrous sulfate•7 hydrate 0.05% and washing the cells obtained with a 0.05M phosphate buffer. The cells were dispersed in a 0.05M phosphate buffer (pH 7.7) at a dark place to prepare a dispersion of the cells having a concentration of 17.3 g dried cells/liter. The resulting cell suspension was divided into two portions. One portion thereof was allowed to stand at 0°C for 1 hour under irradiation at light energy of $5x10^{-1}$ μmol of photons /g cells•second, using a 300 W mercury lamp (supplied by Tokyo Shibaura Denki K.K., Japan), hereinafter this sample being referred to as "light standing". The other portion thereof was allowed to stand at 0 ° C at a dark place for 2 days, hereinafter this sample being referred to as "dark standing".

By using each of the cell suspension, acrylic acid was produced from acrylonitrile and the reaction velocity was studied in view of the amounts of acrylic acid thus produced.

In the case of the light standing bacteria, a mixture of the cells 0.1 part (herein the quantity being shown by the weight of dried cells), acrylonitrile 2.5 part and 0.05 M phosphate buffer (pH 7.7) 97.4 parts was subjected to reaction at 0 ° C in a 50 ml glass reactor under stirring for 120 minutes under the same irradiation condition as described above, hereinafter this reaction being referred to as "irradiated reaction". In the case of the dark standing bacteria, the above mentioned reaction was repeated except that light was not applied, hereinafter this reaction being referred to as "dark reaction". After the termination of the reaction, acrylic acid contained in each of the reaction-liquor whose reaction had been terminated was subjected to quantitative analysis by high performance liquid chromatography. As a result, the amount of acrylic acid (hereinafter referred to as Aa) per unit bacterial cells was 1950 μ mol Aa/mg cells•minute in the light standing-irradiated reaction, and 127 μ mol Aa/mg cells•minute in the dark standing-dark reaction,

respectively. The ratio of these production performances was light standing-light irradiated reaction/dark standing-dark reaction = 15.4.

In the following examples, the value of 1950 μ mol Aa/mg cells•minute obtained by the light standing-irradiated reaction of Example 1 is shown by a relative value of 100U. Thus, the value obtained by the dark standing-dark reaction of Example 1 amounts to 6.5U.

The light standing-light irradiated reaction and the dark standing-dark reaction were repeated in completely the same way as described above except that acrylamide instead of acrylonitrile was used as a substrate. As a result, no substantial difference was seen in the velocity of the reaction to produce acrylic acid.

Examples 2 through 14

The reaction liquor having the same composition as that of Example 1 was prepared by using a variety of nitrile compounds and each of the light standing and dark standing bacterial cells prepared as in Example 1. The reaction liquor was subjectd to reaction as in Example 1. The corresponding organic acids contained in the reaction-terminated liquor were subjected to quantitative analysis by high performance liquid chromatography to obtain the results shown in Table 1, respectively.

Table 1

| Example | Starting nitrile compound | Organic acid produced | Light standing-irradiated reaction(U) | Dark standing-dark reaction (U) | Light standing-irradiated reaction/dark standing-dark reaction |
|---|---|---|---|---|---|
| 2 | acetonitrile | acetic acid | 26.2 | 1.6 | 16.2 |
| 3 | propionitrile | propionic acid | 108.5 | 7.2 | 15.0 |
| 4 | n-butyronitile | butyric acid | 150.6 | 8.1 | 18.6 |
| 5 | methacrylonitrile | methacrylic acid | 101.2 | 4.9 | 20.5 |
| 6 | cyanopyridine | nicotinic acid | 18.1 | 2.1 | 8.6 |
| 7 | malononitrile | malonic acid | 86.2 | 5.1 | 16.8 |
| 8 | succinonitrile | succinic acid | 226.2 | 10.2 | 22.2 |
| 9 | lactonitrile | DL-lactic acid | 49.3 | 3.8 | 13.0 |
| 10 | aminoacetonitrile | glycine | 68.0 | 4.1 | 16.5 |
| 11 | α-aminopropionitrile | DL-alanine | 82.9 | 5.1 | 15.3 |
| 12 | β-aminopropionitrile | β-alanine | 56.4 | 3.5 | 16.2 |
| 13 | α-aminophenylpropionitrile | DL-phenylalanine | 39.5 | 3.8 | 10.5 |
| 14 | α-aminomethylthio-butyronitrile | DL-methionine | 45.5 | 2.8 | 16.3 |

Examples 15 through 19

Washed bacterial cells were obtained by preparing as in Example 1 the strains of the following genuses: Bacillus (CBS-494), Bacteridium (CBS-496), Micrococcus (CBS-497), Brevibacterium (CBS-717), and Nocardia (N-775). The strains having CBS numbers are those deposited at and obtained from Centraal Bureau voor Schimmelcultures (CBS) at Osterstraat 1, Baarn, Netherlands. The washed cells were treated as in Example 1 to obtain light-standing and dark-standing cells. By using the cells in the following formulation, organic acid was produced from acrylonitrile and the reaction velocity was studied in view of the amounts of organic acid thus produced.

In the case of the light-standing strains, a mixture of cells 0.1 part, acrylonitrile 2.5 part and 0.05 M phosphate buffer (pH 7.7) 97.4 parts was subjected to reaction under stirring at 0°C for 120 minutes (irradiated reaction). In the case of the dark-standing strains, the reaction was repeated except that light was not applied (dark reaction). Acrylic acid contained in each of the reaction-terminated liquor was subjected to quantitative analysis by high performance liquid chromatography to obtain the results shown in Table 2.

Table 2

| Example | Genus of bacteria used | Strain | Light standing-light irradiation reaction (U) | Dark standing-dark reaction (U) | The light reaction/the dark reaction |
|---|---|---|---|---|---|
| 15 | Bacillus | CBS-494 | 60.8 | 7.3 | 8.3 |
| 16 | Bacteridium | CBS-496 | 82.5 | 7.9 | 10.4 |
| 17 | Micrococcus | CBS-497 | 53.4 | 5.6 | 9.5 |
| 18 | Brevibacterium | CBS-717 | 107.2 | 6.9 | 15.5 |
| 19 | Nocardia | N-775 | 28.0 | 2.4 | 11.6 |

Examples 20 through 24

5ml each of dark standing cell suspension of N-774 strain obtained in Example 1 and 20 ml each of 0.05 M phosphate buffer (pH 7.7) were taken into 50 ml steel vessels. The resulting cell suspension was

irradiated with a blacklight (Model FL-4BL-G, Tokyo Shibaura Denki K.K.) at 0°C for 1 hour, wherein the light energy was controlled as shown in Table 3 by use of a filter having different optical areas placed at an upper portion of the vessel.

To each of the cell suspension irradiated with light was added 25 ml of a 5% acrylonitrile solution (in 0.05 mol phosphate buffer solution, pH 7.7). The mixture was subjected to reaction at 0°C for 120 minutes under the same condition of light irradiation.

The quantity of organic acid contained in these reaction liquors was subjected to quantitative analysis by high performance liquid chromatography to obtain a reaction velocity. The results are shown in Table 3.

For comparison, reactions were carried out in the same way using the liquid extract which was obtained by the treatment of N-774 strain using French press in accordance with a conventional method. The results are shown in Table 3.

Table 3

| Example | Light energy applied (μE/g cells•sec.) | Reaction velocity after irradiation for 1 hour (U) |
|---|---|---|
| 20 | no irradiation | 6.5 |
| | | 6.5 |
| 21 | $1x10^{-3}$ | 15.5 |
| | | 16.2 |
| 22 | $2x10^{-3}$ | 25.2 |
| | | 27.8 |
| 23 | $1x10^{-2}$ | 45.2 |
| | | 43.6 |
| 24 | $1x10^{-1}$ | 75.5 |
| | | 76.6 |
| Note: The lower figure in each row is for liquid extracts from cells. | | |

Examples 25 through 29

5 ml each of dark standing cell suspension of N-774 strain obtained in Example 1 and 20 ml each of 0.05 M phosphate buffer (pH 7.7) were taken into 50 ml steel vessels. The resulting cell suspension was irradiated with a daylight lamp (supplied by Tokyo Shibaura Denki K.K.) at 0°C under stirring, wherein the portion of light in a wavelength up to 430 nm was removed by the use of colored glass filters (Model Y45, supplied by Tokyo Shibaura Denki K.K.) each having the same optical area placed at an upper portion of the vessel and the light energy was controlled as shown in Table 4 by changing the irradiation time for each of the cell suspensions.

To each of the cell suspension irradiated with light was added 25 ml of a 5% acrylonitrile solution (in 0.05 mol phosphate buffer, pH 7.7). The mixture was subjected to reaction at 0°C for 120 minutes under the same condition of light irradiation.

The quantity of organic acid contained in these reaction liquors was subjected to quantitative analysis by high performance liquid chromatography to obtain a reaction velocity. The results are shown in Table 4.

Table 4

| Example | Light energy applied (μE/g cells•second) | Reaction velocity (U) |
|---|---|---|
| 25 | $1x10^{-3}$ | 9.8 |
| 26 | $2x10^{-3}$ | 12.5 |
| 27 | $5x10^{-3}$ | 20.3 |
| 28 | $1x10^{-2}$ | 35.5 |
| 29 | $1x10^{-1}$ | 48.5 |

Example 30

Washed bacterial cells of N-774 strain obtained by culturing the strain in the way as in Example 1 were dispersed in physiological saline to prepare a suspension of cells having a concentration of 2%. 800 ml of the cell suspension was placed in a separable glass flask and was subjected to reaction at 10°C under stirring by gradually adding 65g of acrylonitrile while being allowed to accept light energy of about 500 μmol of photons/g cells•second by irradiation with a blacklight lamp (Model FL-4BL-G, supplied by Tokyo Shibaura Denki K.K.), wherein the pH was controlled with sodium hydroxide solution and the concentration of acrylonitrile in the reaction system was controlled up to 2%. In the above reaction of producing acrylic acid from acrylonitrile, the pH of the reaction system was 8.0 for the first 2 hours and then was lowered to 6.5 to 7.0 for the later 5 hours. The reaction-terminated liquor contained 10% (w/w) of acrylic acid and most of nitrile was converted into the acid.

For comparison, the reaction was repeated using the dark standing cells and by dark reaction. As a result, the reaction liquor contained not more than 1% (w/w) of acrylic acid 10 hours after starting the reaction.

## Claims

**1.** A process for hydrating and hydrolyzing a nitrile compound by the action of a microorganism having nitrilase activity to convert the nitrile compound into the corresponding organic acid which comprises conducting the reaction under the conditions wherein:
   (a) the microorganism having nitrilase activity is positive Gram staining;
   (b) the microbial cells are allowed to accept light energy of at least about $2x10^{-3}$ μmol of photons/g microbial cells second before termination of the hydration reaction; and
   (c) the hydration reaction is carried out on an industrial scale in a vessel composed at least partly of a material which does not permit transmission therethrough of light.

**2.** The process according to Claim 1, in which the light has a wavelength of 100 to 800 nm.

**3.** The process according to any of Claims 1 to 2, in which the light energy is at least about $5x10^{-3}$ μmol of photons/g microbial cells second.

**4.** The process according to any of Claims 1 to 3, in which the irradiation with light is carried out before and/or after contacting the microorganism with the nitrile compound.

**5.** The process according to any of Claims 1 to 4, in which the microorganism belongs to the genus selected from the group consisting of Corynebacterium, Nocardia, Bacillus, Bacteridium, Micrococcus, and Brevibacterium.

**6.** The process according to any of Claims 1 to 5, in which the nitrile compound is selected from the group consisting of acetonitrile, propionitrile, n-butyronitrile, isobutyronitrile, acrylonitrile, methacrylonitrile, benzonitrile, cyanopyridine, malononitrile, succinonitrile, fumaronitrile, lactonitrile, β-hydroxypropionitrile, aminoacetonitrile, α-aminopropionitrile, β-aminopropionitrile, α-aminophenylpropionitrile, and α-aminomethylthiobutylonitrile.

## Patentansprüche

1. Verfahren zum Hydratisieren und Hydrolysieren einer Nitrilverbindung durch Einwirkung eines Mikroorganismus mit Nitrilase-Aktivität unter Umwandlung der Nitrilverbindung in die entsprechende organische Säure, bei dem man die Umsetzung unter den folgenden Bidingungen durchfuhrt:
   (a) Der Mikroorganismus mit Nitrilaseaktivität ist ein solcher mit positiver Gramfärbung;
   (b) Man läßt die Zellen des Mikroorganismus Lichtenergie von mindestens etwa 2 x $10^{-3}$ µmol Photonen/g Zellen des Mikroorganismus mal Sekunden vor der Beendigung der Hydratisierungsreaktion aufnehmen; und
   (c) Die Hydratisierungsreaktion wird in großtechnischem Maßstab in einem Gefäß durchgeführt, das mindestens teilweise aus einem nicht lichtdurchlässigen Material besteht.

2. Verfahren gemäß Anspruch 1, wobei das Licht eine Wellenlänge von 100 bis 800 nm besitzt.

3. Verfahren gemäß Anspruch 1 oder 2, wobei die Lichtenergie mindestens etwa 5 x $10^{-3}$ µmol Photonen/g Zellen des Mikroorganismus mal Sekunden beträgt.

4. Verfahren gemäß einem der Ansprüche 1 bis 3, wobei die Bestrahlung mit Licht vor und bzw. oder nach Inkontaktbringen des Mikroorganismus mit der Nitrilverbindung durchgeführt wird.

5. Verfahren gemäß einem der Ansprüche 1 bis 4, wobei der Mikroorganismus zu der Gattung Corynebacterium, Nocardia, Bacillus, Bacteridium, Micrococcus oder Brevibacterium gehört.

6. Verfahren gemäß einem der Ansprüche 1 bis 5, wobei die Nitrilverbindung aus der folgenden Gruppe ausgewählt ist: Acetonitril, Propionitril, n-Butyronitril, Isobutyronitril, Acrylnitril, Methacrylnitril, Benzonitril, Cyanopyridin, Malononitril, Succinonitril, Fumaronitril, Lactonitril, β-Hydroxypropionitril, Aminoacetonitril, α-Aminopropionitril, β-Aminopropionitril, α-Aminophenylpropionitril und α-Aminomethylthiobutyronitril.

## Revendications

1. Procédé pour hydrater et hydrolyser un nitrile par l'action d'un microorganisme doué d'activité nitrilasique afin de convertir le nitrile en l'acide organique correspondant, qui consiste à conduire la réaction dans des conditions dans lesquelles :
   (a) le microorganisme doué d'activité nitrilasique est positif à la coloration de Gram ;
   (b) les cellules microbiennes recoivent une énergie lumineuse d'au moins environ 2 × $10^{-3}$ µmol de photons/g de cellules microbiennes.seconde avant la fin de la réaction d'hydratation ; et
   (c) la réaction d'hydratation est conduite à une échelle industrielle dans un récipient constitué au moins en partie d'un matériau qui ne permet pas le passage de la lumière.

2. Procédé selon la revendication 1, dans lequel la lumière a une longueur d'onde de 100 à 800 nm.

3. Procédé selon l'une quelconque des revendications 1 et 2, dans lequel l'énergie lumineuse est d'au moins environ 5 × $10^{-3}$ µmol de photons/g de cellules microbiennes.seconde.

4. Procédé selon l'une quelconque des revendications 1 à 3, dans lequel l'irradiation par la lumière est effectuée avant et/ou après la mise en contact du microorganisme avec le nitrile.

5. Procédé selon l'une quelconque des revendications 1 à 4, dans lequel le microorganisme appartient à un genre choisi dans le groupe formé par *Corynebacterium*, *Nocardia, Bacillus, Bactecidium, Micrococcus* et *Brevibacterium.*

6. Procédé selon l'une quelconque des revendications 1 à 5, dans lequel le nitrile est choisi dans le groupe formé par l'acétonitrile, le propionitrile, le *n*-butyronitrile, l'isobutyronitrile, l'acrylonitrile, le méthacrylonitrile, le benzonitrile, la cyanopyridine, le malononitrile, le succinonitrile, le fumaronitrile, le lactonitrile, le β-hydroxypropionitrile, l'aminoacétonitrile, le α-aminopropionitrile, le β-aminopropionitrile, le α-aminophénylpropionitrile et le α-aminométhylthiobutyronitrile.